# EUROPEAN PATENT SPECIFICATION

(11) **EP 1 363 591 B1**
(45) Date of publication and mention of the grant of the patent: **13.06.2007**
(21) Application number: 01948812.1
(22) Date of filing: 27.06.2001
(51) Int. Cl.: A61K 8/23, A61K 8/90, A61Q 5/12

(54) **ANHYDROUS SELF-HEATING COSMETIC COMPOSITIONS, ESPECIALLY ANHYDROUS HAIR CONDITIONING COMPOSITIONS**
WASSERFREIE SELBSTERHITZENDE ZUSAMMENSETZUNGEN, INSBESONDERE WASSERFREIE HAARKONDITIONIERMITTEL
COMPOSITIONS COSMETIQUES AUTO-CHAUFFANTES ANHYDRES, ET PLUS PARTICULIEREMENT COMPOSITIONS D'APRES-SHAMPOOING ANHYDRES

(30) Priority: 01.02.2001 US 103422
(43) Date of publication of application: 26.11.2003
(73) Proprietor: THE PROCTER & GAMBLE COMPANY, Cincinnati, Ohio 45202 (US)
(72) Inventor: UCHIDA, Mikio, Ashiya-shi, Hyogo 659-0025 (JP); MITSUMATSU, Arata, Nishinomiya, Hyogo 662-0018 (JP); YASUFUKU, Keiichi, Kobe, Hyogo 658-0046 (JP); AZUMA, Misa, Osaka 581-0843 (JP)
(74) Representative: Kohol, Sonia
(86) International application number: PCT/US2001/020651
(87) International publication number: WO 2002/060407

(56) References cited:
- EP-A- 0 586 929
- EP-A- 0 897 719
- WO-A-00/38621
- WO-A-86/05389
- US-A- 3 723 324

## Description

### TECHNICAL FIELD

The present invention relates to an anhydrous cosmetic composition which warms by mixing with water.

### BACKGROUND

A variety of cosmetic products such as hair care products and skin care products have been used to the hair and/or skin. With respect to hair care products, for example, hair shampoo products are used for cleansing the hair by removing excess soil and sebum; hair conditioning products are used for providing various conditioning benefits such as moisturized feel, softness, and static control to the hair; hair styling products are used for setting hair style and/or maintaining hair style; hair color products are used for changing hair color and/or maintaining hair color; and hair growth products are used for encouraging hair growth.

The efficacy of cosmetic products such as hair care products and skin care products are changed by various factors, for example, amount of products applied, how long products are applied on the hair, temperatures of products, the way of applying products to the hair, and so on. Thus, it may not be easy to obtain expected efficacy from cosmetic products such as hair care products and skin care products.

A variety of approaches have been developed to obtain expected efficacy from cosmetic products. For example, inorganic salts and/or oxides which generate a heat by mixing with water are contained in anhydrous cosmetic compositions, being expected to improve penetration and deposition of conditioning components to skin and/or hair by its heat generating (described in Japanese Patent Laid-open H11-228332). However, it has been found that; inorganic heat generating agents such as inorganic salts and/or oxides quickly agglomerate by presence of water, even small amount of water, and form larger clods which cause uncomfortable gritty feel to the skin and /or hair. Agglomerations may form before and/or during applying the compositions containing inorganic heat generating agents on the skin and/or hair, and may also form during storage.

Based on the foregoing, there remains a desire for obtaining enhanced efficacy from cosmetic products such as hair care products and skin care products, i.e., a desire for obtaining improved benefits from cosmetic products. With respect to hair care products, for example, there remains a desire for obtaining improved cleansing benefits from hair shampoo products, and obtaining improved hair conditioning benefits such as moisturized feel, softness, and static control from hair conditioning products, while reducing gritty feel to the hair, hair scalp, and/or hands.

None of the existing art provides all of the advantages and benefits of the present invention.

### SUMMARY

The present invention is directed to an anhydrous cosmetic composition comprising:
(a) an inorganic heat generating agent which generates a heat by mixing with water; and
(b) a polyoxyalkylene derivative according to claim 1.

The present invention provides anhydrous cosmetic compositions which warm by mixing with water. The compositions of the present invention can provide enhanced efficacy while reducing gritty feel to the skin and/or hair. It is believed that; warming cosmetic compositions such as hair care compositions and skin care compositions can provide enhanced efficacy, i.e., can provide improved benefits. With respect to hair care compositions, for example, it is believed that; warming hair shampoo compositions can provide improved cleansing benefits, warming hair styling compositions can provide improved styling benefits, warming hair conditioning compositions can provide improved hair conditioning benefits due to improved penetration of ingredients, and warming hair color compositions and warming hair growth compositions can also provide improved benefits. With respect to skin care compositions, for example, it is believed that; warming body shampoo compositions can provide improved cleansing benefits, warming face cleansing compositions can provide improved cleansing benefits, warming skin conditioning compositions can provide improved conditioning benefits, and warming shaving compositions can provide improved shaving benefits. It is also believed that; polyoxyalkylene derivatives can help the dispersion of inorganic heat generating agents in inert carriers, thus, prevent the agglomeration of inorganic heat generating agents which causes gritty feel to the skin and/or hair. It is also believed that; some of the polyoxyalkylene derivatives can provide slippery feel which eases gritty feel caused by inorganic heat generating agents.

### DETAILED DESCRIPTION

While the specification concludes with claims particularly pointing out and distinctly claiming the invention, it is believed the present invention will be better understood from the following description.

All percentages are by weight of the total composition unless otherwise indicated. All ratios are weight ratios unless otherwise indicated. All percentages, ratios, and levels of ingredients referred to herein are based on the actual amount of the ingredient, and do not include solvents, fillers, or other materials with which the ingredient may be combined as commercially available products, unless otherwise indicated.

As used herein, "comprising" means that other steps and other ingredients which do not affect the end result can be added. This term encompasses the terms "consisting of and "consisting essentially of",

All cited references are incorporated herein by reference in their entireties. Citation of any reference is not an admission regarding any determination as to its availability as prior art to the claimed invention.

### ANHYDROUS COSMETIC COMPOSITIONS

The anhydrous cosmetic compositions of the present invention warm by a heat from heat generating agents when mixing with water. As used in the present invention, "anhydrous" means that the compositions contain 5% or less of water. The anhydrous compositions of the present invention contain, preferably 3% or less, more preferably 1% or less, still more preferably substantially free of water. The anhydrous cosmetic compositions warm to a temperature of, preferably from about 25°C to about 80°C, more preferably from about 30°C to about 60°C, still more preferably from about 35°C to about 45°C. This temperature can be adjusted by, for example, choosing the heat generating agents, the amount of the heat generating agent, and additional agents which can control the heat generating reaction.

Various anhydrous cosmetic compositions such as anhydrous hair care compositions and anhydrous skin care compositions can be used in the present invention. The anhydrous hair care compositions useful herein include, for example, anhydrous hair shampoo compositions, anhydrous hair styling compositions, anhydrous hair conditioning compositions, anhydrous hair color compositions, anhydrous hair growth compositions, and mixtures thereof. The anhydrous skin care compositions useful herein include, for example, anhydrous body shampoo compositions, anhydrous face cleansing compositions, anhydrous skin conditioning compositions, anhydrous shaving compositions, and mixtures thereof.

It is believed that; warming cosmetic compositions such as hair care compositions and skin care compositions can provide enhanced efficacy, i.e., can provide improved benefits. With respect to hair care compositions, for example, it is believed that; warming hair shampoo compositions can provide improved cleansing benefits, warming hair styling compositions can provide improved styling benefits, warming hair conditioning compositions can provide improved hair conditioning benefits due to improved penetration of ingredients, warming hair color compositions and warming hair growth compositions can also provide improved benefits. With respect to skin care compositions, for example, it is believed that; warming body shampoo compositions can provide improved cleansing benefits, warming face cleansing compositions -can provide improved cleansing benefits, warming skin conditioning compositions can provide improved conditioning benefits, and warming shaving compositions can provide improved shaving benefits.

The anhydrous cosmetic compositions of the present invention can be in the form of rinse-off products or leave-on products, can be transparent or opaque, and can be formulated in a wide variety of product forms, including but not limited to lotions, creams, gels, emulsions, mousses, and sprays.

The anhydrous cosmetic compositions of the present invention can be mixed with water and applied to the hair and/or skin by any conventional method well known in the art. For example, the anhydrous compositions can be applied to hair and/or skin after mixing with water on hands and/or in a certain vessel. The anhydrous compositions can be applied to wet hair and/or wet skin to mix with water remaining on the hair and/or skin. The anhydrous compositions can be applied to wet and/or dry hair and/or skin to mix with water when rinsed-off. INORGANIC HEAT GENERATING AGENT

The anhydrous cosmetic compositions of the present invention comprise an inorganic heat generating agent which generates a heat by mixing with water.

The anhydrous inorganic salts are sodium sulfate (Na₂SO₄), calcium sulfate (CaSO₄), magnesium sulfate (MgSO₄), aluminum sulfate (Al(SO₄)₃), calcium chloride (CaCl₂), magnesium chloride(MgCl₂), calcium oxide (CaO), and mixtures thereof, in view of their effective heat generation, mildness to hair and/or skin, and easy handling. More preferred is anhydrous magnesium sulfate (MgSO₄).

The inorganic heat generating agents useful herein have an average diameter of, preferably from about 0.01 µm to about 40µm, more preferably from about 0.05µm to about 30µm, still more preferably from about 0.1 µm to about 20µm, in view of preventing gritty feel.

The inorganic heat generating agent is included in the compositions at a level by weight of, preferably from about 5% to about 60%, more preferably from about 15% to about 50%, still more preferably from about 25% to about 45%. POLYOXYALKYLENE DERIVATIVES

The anhydrous cosmetic composition of the present invention comprises polyoxyalkylene derivatives. It is believed that; polyoxyalkylene derivatives can help the dispersion of inorganic heat generating agents in inert carriers, thus, prevent the agglomeration of inorganic heat generating agents which causes gritty feel to the skin and /or hair. It is also believed that; some of the polyoxyalkylene derivatives can provide slippery feel which eases gritty feel caused by inorganic heat generating agents.

The polyoxyalkylene derivatives are polyoxyethylene/polyoxypropylene copolymer, polyoxyethylene glyceryl ester, and mixtures thereof. The polyoxyalkylene derivatives useful herein are preferably water soluble polyoxyalkylene derivatives. Among them, polyoxyethylene/polyoxypropylene copolymers are used in view of preventing agglomeration of inorganic heat generating agents, and polyoxyethylene glyceryl esters are used in view of providing slippery feel.

When the polyoxyalkylene derivative is used in view of preventing agglomeration of inorganic heat generating agents, the polyoxyalkylene derivative is included in the compositions at a level by weight of, from about 0.1% to about 10%, more preferably from about 0.5% to about 10%, still more preferably from about 1% to about 5%.

When the polyalkylene derivative is used in view of providing slippery feel, the polyoxyalkylene derivative is included in the compositions at a level by weight of, from about 10% to about 90%, more preferably from about 15% to about 85%, still more preferably from about 20% to about 80%.

Preferred polyoxyethylene glyceryl esters include, for example, following (i) and (ii).
(i) PEG-modified glycerides having the structure: 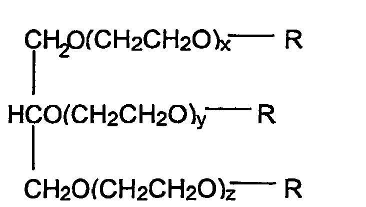 wherein one or more of the R groups is selected from saturated or unsaturated fatty acid moieties derived from animal or vegetable oils such as palmitic acid, lauric acid, oleic acid or linoleic acid wherein the fatty acid moieties have a carbon length chain of from 12 and 22, any other R groups are hydrogen, x, y, z are independently zero or more, the average sum of x+y+z (the degree of ethoxylation) is equal to from about 10 to about 45. Preferably, the PEG-modified glycerides have an HLB value of about 20 or less, more preferably about 15 or less, still preferably about 11 or less. Preferably, the PEG-modified glycerides have from 2 to 3 fatty acid R groups, more preferred have 3 fatty acid R groups (PEG-modified triglycerides). Preferably, the average sum of x+y+z (the degree of ethoxylation) is equal to from about 20 to 30, more preferred is an average sum of 25. Most preferred are PEG-substituted triglycerides having 3 oleic acid R groups, wherein the average degree of ethoxylation is about 25 (PEG-25 glyceryl trioleate). Preferred commercially available PEG-modified triglycerides include Tagat TO ®, Tegosoft GC, Tagat BL 276®, (all three manufactured by Goldschmidt Chemical Corporation) and Crovol A-40, Crovol M-40 (manufactured by Croda Corporation). Other preferred commercially available PEG-modified triglycerides include Tagat S ® and Tagat S 2 ® (manufactured by Goldschmidt Chemical Corporation).
(ii) PEG-modified glyceryl fatty acid esters having the structure: 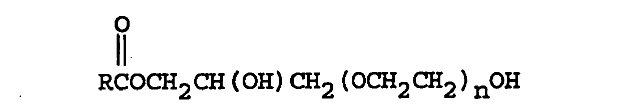 wherein n, the degree of ethoxylation, is from about 4 to about 200, preferably from about 5 to about 150, more preferably from about 20 to about 120, and wherein R comprises an aliphatic radical having from about 5 to about 25 carbon atoms, preferably from about 7 to about 20 carbon atoms. Suitable polyethylene glycol derivatives of glycerides can be polyethylene glycol derivatives of hydrogenated castor oil. For example, PEG-20 hydrogenated castor oil, PEG-30 hydrogenated castor oil, PEG-40 hydrogenated castor oil, PEG-45 hydrogenated castor oil, PEG-50 hydrogenated castor oil, PEG-54 hydrogenated castor oil, PEG-55 hydrogenated castor oil, PEG-60 hydrogenated castor oil, PEG-80 hydrogenated castor oil, and PEG-100 hydrogenated castor oil. Preferred for use in the compositions herein is PEG-60 hydrogenated castor oil. Other suitable polyethylene glycol derivatives of glycerides can be polyethylene glycol derivatives of stearic acid, for example, PEG-30 stearate, PEG-40 stearate, PEG-50 stearate, PEG-75 stearate, PEG-90 stearate, PEG-100 stearate, PEG-120 stearate, and PEG-150 stearate. Preferred for use in the compositions herein is PEG-100 stearate.

Preferred polyoxyethylene/polyoxypropylene copolymers include, for example, polyoxyethylene/polyoxypropylene random copolymer and polyoxyethylene/polyoxypropylene block copolymer.

Among these polyoxyalkylene derivatives, polyoxyethylene/polyoxypropylene copolymers including polyoxyethylene/polyoxypropylene random copolymer and polyoxyethylene/polyoxypropylene block copolymer are preferably used in the composition of the present invention in view of their suspending benefit. More preferred is polyoxyethylene/polyoxypropylene block copolymer, still more preferred is polyoxyethylene/polyoxypropylene block copolymer having a weight ratio of polyoxyethylene to polyoxypropylene of from about 5:10 to about 8:10, even more preferred is the block copolymer having the ratio of 8:10.

Commercially available polyoxyalkylene derivatives useful herein include: polyoxyethylene/polyoxypropylene block copolymer, having CTFA name Poloxamer 338, available from BASF under trade name Pluronic F-108, and also available from Sanyo Chemical under trade name Newpol PE-108; and having CTFA name Poloxamer 288, available from BASF under trade name Pluronic F-98, and also available from Sanyo Chemical under trade name Newpol PE-98.

### INERT CARRIER

The anhydrous cosmetic composition of the present invention preferably comprises an inert carrier.

The inert carrier is included in Composition A at a level by weight of, preferably from about 10% to about 90%, more preferably from about 25% to about 90%, still more preferably from about 30% to about 85%.

The inert carrier can be included in Composition B at a level by weight of, preferably from about 3% to about 85%, more preferably from about 5% to about 80%, still more preferably from about 10% to about 70%.

The inert carriers useful herein are liquid carriers and include; for example, liquid polyhydric alcohols such as polyethylene glycol, polypropylene glycol, 1,2-propane diol or propylene glycol, 1,3-propane diol, hexylene glycol, glycerin, diethylene glycol, dipropylene glycol, 1,2-butylene glycol, 1,4-butylene glycol; liquid paraffin; mineral oil; vegetable oil; low melting point oil such as pentaerythritol tetraisostearate; and mixtures thereof. The liquid polyhydric alcohols such as polyethylene glycol can also be used as "ADDITIONAL HEAT GENERATING AGENT" described below. The low melting point oil useful herein is described below as a conditioning agent under the title "LOW MELTING POINT OIL". Preferred are polyethylene glycol, polypropylene glycol, glycerin, liquid paraffin, mineral oil, vegetable oil, pentaerythritol tetraisostearate, and mixtures thereof in view of physical properties such as viscosity and fluidity. More preferred is polyethylene glycol in view of its ability to generate a heat by mixing with water and physical properties such as viscosity and fluidity.

The polyethylene glycols useful herein are those having the formula:

H(OCH₂CH₂)ₙ -OH

wherein n has an average value of from 4 to 12.

The polyethylene glycol described above is also known as a polyethylene oxide, and polyoxyethylene. Polyethylene glycols useful herein that are especially preferred are PEG-200 wherein n has an average value of about 4. Commercially available preferred polyethylene glycol includes, for example, PEG-200 having trade name Carbowax PEG-200 available from Union Carbide).

### REACTION CONTROL AGENTS

The anhydrous cosmetic compositions of the present invention preferably contain reaction control agents which can control the heat generating reaction of the inorganic heat generating agent. The reaction control agents may slow down the reaction, or accelerate the reaction. The reaction control agents may also control the temperature to which the cosmetic composition warms up.

Acids can be used as reaction control agents for accelerating the reaction of the inorganic heat generating agents. The acid useful herein includes, for example, citric acid, sodium diphosphate, potassium diphophate, ℓ-glutamic acid, lactic acid, hydrochloric acid, malic acid, succinic acid, acetic add, fumaric acid, ℓ-glutamic acid hydrochloride, tartaric acid, and mixtures thereof; preferably ℓ-glutamic acid, lactic acid, hydrochloric acid, and mixtures thereof. Among the above acids, citric acid is preferably used herein. Some acids can also be used together with amidoamines for providing conditioning benefits as described below. The acid can be contained at a level such that the mole ratio of the inorganic heat generating agent to acid is from about 1:0.1 to about 1:10, preferably from about 1:0.5 to about 1:5.

Water absorbing polymer can be used as reaction control agents for slowing down the reaction of the inorganic heat generating agent. The water absorbing polymer useful herein includes, for example, vinyl polymers such as cross linked acrylic acid polymers with the CTFA name Carbomer, carboxylic acid/carboxylate copolymers such as acrylic acid/alkyl acrylate copolymers with the CTFA name Acrylates/C1a-30 Alkyl Acrylate Crosspolymer, cellulose derivatives and modified cellulose polymers such as Hydroxyethylcellulose and Hydroxypropyl cellulose, polyvinylpyrrolidone, polyvinyl alcohol, guar gum, other gums, starch-based polymers, alginic acid-based polymers, acrylate polymers, polyalkylene glycols having a molecular weight of more than about 1000, and mixtures thereof. These water absorbing polymers can also be used as the "VISCOSITY MODIFYING AGENT" described below. Among the above water absorbing polymers, preferred are cellulose derivatives and modified cellulose polymers, and more preferred is Hydroxyethylcellulose. The water absorbing polymers can be included in the compositions at a level by weight of, preferably from about 0.2% to about 20%, more preferably from about 0.5% to about 15%, still more preferably from about 1% to about 10%.

### HEAT RESERVING MATERIALS

The anhydrous cosmetic compositions of the present invention may contain heat reserving materials which can reserve a heat. The heat reserving material can be used for prolonging heating, and may be used for slowing down the warming speed, and may also control the temperature to which the cosmetic composition warms up.

The heat reserving materials include, for example, silica gel, carboxymethyl cellulose gel, phase-changing materials, and mixtures thereof. The phase-changing materials useful herein are those which have a melting point of from about 25°C to about 80°C. The phase-changing materials useful herein include, for example, a fatty compound such as fatty alcohol and fatty acid; hydrocarbons; a mixture of hydrocarbons and foamed polyolefin; and mixtures thereof. Fatty compound useful herein are disclosed below under the title "HIGH

### MELTING POINT FATTY COMPOUND".

The heat reserving material can be included in the compositions at a level by weight of, preferably from about 0.2% to about 20%, more preferably from about 0.5% to about 15% still more preferably from about 1 % to about 10%.

### VISCOSITY MODIFYING AGENT

The anhydrous cosmetic composition of the present invention may contain a viscosity modifying agent. The viscosity modifying agent useful herein includes, for example, vinyl polymers such as cross linked acrylic acid polymers with the CTFA name Carbomer, carboxylic acid/carboxylate copolymers such as acrylic acid/alkyl acrylate copolymers with the CTFA name Acrylates/C10-30 Alkyl Acrylate Crosspolymer, cellulose derivatives and modified cellulose polymers, polyvinylpyrrolidone, polyvinyl alcohol, guar gum, other gums, starch-based polymers, alginic acid-based polymers, acrylate polymers, polyalkylene glycols having a molecular weight of more than about 1000, inorganic water soluble material such as bentonite, aluminum magnesium silicate, laponite, hectorite, and anhydrous silicic acid, and mixtures thereof. The polymers described herein can also be used as the "VISCOSITY MODIFYING AGENT" described above. Some polyalkylene glycols described herein can also be used as hair conditioning agents described below under the title "POLYPROPYLENE GLYCOL" and "POLYETHYLENE GLYCOL".

The viscosity modifying agent can be included in the compositions at a level by weight of, preferably from about 0.01% to about 5%, more preferably from about 0.05% to about 3% still more preferably from about 0.1% to about 3%.

### ADDITIONAL HEAT GENERATING AGENTS

The anhydrous cosmetic compositions of the present invention may contain additional heat generating agents, in addition to the inorganic heat generating agents, which generate a heat by mixing with water. Such additional heat generating agents useful herein include, for example, organic heat generating agents such as polyhydric alcohols.

The polyhydric alcohol useful herein includes, for example, polyethylene glycol, polypropylene glycol, 1,2-propane diol or propylene glycol, 1,3-propane diol, hexylene glycol, glycerin, diethylene glycol, dipropylene glycol, 1,2-butylene glycol, 1,4-butylene glycol, and mixtures thereof. These polyhydric alcohols can also be used as the "INERT CARRIER" described above.

Such additional heat generating agents can be included in the compositions at a level by weight of, preferably from about 2% to about 85%, more preferably from about 5% to about 85%, still more preferably from about 10% to about 85%.

### HAIR CONDITIONING COMPOSITION

The anhydrous cosmetic compositions of the present invention are preferably anhydrous hair care compositions, more preferably anhydrous hair conditioning compositions. The anhydrous hair conditioning compositions preferably comprise hair conditioning agents in addition to the above described inorganic heat generating agent, the polyoxyalkylene derivative, and the inert carrier. The hair conditioning agents useful herein include, for example, high melting point fatty compounds, amidoamines, acids, cationic conditioning agents such as cationic surfactants and cationic polymers, low melting point oils, silicone compounds, polypropylene glycol, polyethylene glycol, and mixtures thereof. Among these hair conditioning agents, preferred are high melting point fatty compounds, amidoamines, acids, and mixtures thereof.

### HIGH MELTING POINT FATTY COMPOUND

The hair conditioning composition of the present invention preferably comprises a high melting point fatty compound. The high melting point fatty compound can be used as the phase changing materials described above under the title "HEAT RESERVING MATERIALS".

The high melting point fatty compound useful herein have a melting point of 25°C or higher, and is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

The high melting point fatty compound can be included in the composition at a level by weight of, preferably from about 0.1 % to about 30%, more preferably from about 0.2% to about 25%, still more preferably from about 0.5% to about 15%.

The fatty alcohols useful herein are those having from about 14 to about 30 carbon atoms, preferably from about 16 to about 22 carbon atoms. These fatty alcohols are saturated and can be straight or branched chain alcohols. Nonlimiting examples of fatty alcohols include, cetyl alcohol, stearyl alcohol, behenyl alcohol, and mixtures thereof.

The fatty acids useful herein are those having from about 10 to about 30 carbon atoms, preferably from about 12 to about 22 carbon atoms, and more preferably from about 16 to about 22 carbon atoms. These fatty acids are saturated and can be straight or branched chain acids. Also included are diacids, triacids, and other multiple acids which meet the requirements herein. Also included herein are salts of these fatty acids. Nonlimiting examples of fatty acids include lauric acid, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl others of alkoxylated fatty alcohols, esters of fatty alcohols, fatty acid esters of compounds having esterifiable hydroxy groups, hydroxy-substituted fatty acids, and mixtures thereof. Nonlimiting examples of fatty alcohol derivatives and fatty acid derivatives include materials such as methyl stearyl ether, the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through 10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, *i.e.* a mixture of fatty alcohols containing predominantly cetyl and stearyl alcohol, wherein the numeric designation indicates the number of ethylene glycol moieties present; C₁-C₃₀ alkyl ethers of the ceteth, steareth, and ceteareth compounds just described; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, and mixtures thereof.

High melting point fatty compounds of a single compound of high purity are preferred. Single compounds of pure fatty alcohols selected from the group consisting of pure cetyl alcohol, stearyl alcohol, and behenyl alcohol are highly preferred. By "pure" herein, what is meant is that the compound has a purity of at least about 90%, preferably at least about 95%. These single compounds of high purity provide good rinsability from the hair when the consumer rinses off the composition.

Commercially available high melting point fatty compounds useful herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan), various fatty acids having tradenames NEO-FAT available from Akzo (Chicago Illinois, USA), HYSTRENE available from Witco Corp. (Dublin Ohio, USA), and DERMA available from Vevy (Genova, Italy).

### AMIDOAMINE

The hair conditioning composition of the present invention preferably comprises an amidoamine of the following general formula:

R¹ CONH (CH₂)ₘ N (R²)₂

wherein R¹ is a residue of C₁₁ to C₂₄ fatty acids, R² is a C₁ to C₄ alkyl, and m is an integer from 1 to 4.

The amidoamine can be included in the composition at a level by weight of, preferably from about 0.05% to about 10%, more preferably from about 0.05% to about 8%, still more preferably from about 0.1 % to about 5%.

Preferred amidoamines useful in the present invention includes stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and mixtures thereof; more preferably stearamidopropyldimethylamine, stearamidoethyldiethylamine, and mixtures thereof.

Commercially available amidoamines useful herein include: stearamidopropyldimethylamine having tradename SAPDMA available from Inolex, and tradename Amidoamine MPS available from Nikko.

### ACIDS

The hair conditioning composition of the present invention preferably comprises an acid selected from the group consisting of ℓ-glutamic acid, lactic acid, hydrochloric acid, malic acid, succinic acid, acetic acid, fumaric acid, ℓ-glutamic acid hydrochloride, tartaric acid, and mixtures thereof; preferably ℓ-glutamic acid, lactic acid, hydrochloric acid, and mixtures thereof. The acid described herein can also be used as the "REACTION CONTROL AGENTS" described above. The acid can be contained at a level such that the mole ratio of amidoamine to acid is, preferably from about 1:0.3 to about 1:1, more preferably from about 1:0.5 to about 1:0.9.

Commercially available acids useful herein include: ℓ-Glutamic acid: ℓ-Glutamic acid (cosmetic grade) available from Ajinomoto.

### PREFERRED HAIR CONDITIONING COMPOSITIONS

In one preferred embodiment of the present invention, the anhydrous hair conditioning composition (Conditioning composition A) comprises by weight:
(a) from about 5% to about 60% of the inorganic heat generating agent which generates a heat by mixing with water, preferably anhydrous magnesium sulfate;
(b) from about 0.1 % to about 10% of the polyoxyalkylene derivative comprising polyoxyethylene/polyoxypropylene copolymer, preferably polyoxyethylene/polyoxypropylene block copolymer;
(c) from about 0.1 % to about 30% of the high melting point fatty compound, preferably the high melting point fatty compound selected from the group consisting of cetyl alcohol, stearyl alcohol, behenyl alcohol, and mixtures thereof;
(d) from about 0.05% to about 10% of the amidoamine, preferably, the amidoamine selected from the group consisting of stearamidopropyl dimethylamine, stearamidoethyl diethylamine, and mixtures thereof;
(e) the acid at a level such that the mole ratio of the amidoamine to the acid is from about 1:0.3 to about 1:1, preferably, ℓ-Glutamic acid at a level such that the mole ratio of amidoamine to acid is from about 1:0.5 to about 1:0.9; and
(f) from about 10% to about 90% of the inert carrier, preferably, polyethylene glycol.

In another preferred embodiment of the present invention, the anhydrous hair conditioning composition (Conditioning composition B) comprises by weight
(a) from about 5% to about 60% of the inorganic heat generating agent which generates a heat by mixing with water, preferably anhydrous magnesium sulfate;
(b) from about 10% to about 90% of the polyoxyalkylene derivative comprising polyoxyethylene glyceryl ester,
(c) from about 0.1% to about 30% of a high melting point fatty compound, preferably the high melting point fatty compound selected from the group consisting of cetyl alcohol, stearyl alcohol, behenyl alcohol, and mixtures thereof;
   from about 0.05% to about 10% of an preferably, the amidoamine selected from the group consisting of stearamidopropyl dimethylamine, stearamidoethyl diethylamine, and mixtures thereof; and
(e) an acid selected from the group consisting of ℓ-glutamic acid, lactic acid, hydrochloric acid, malic acid, succinic acid, acetic acid, fumaric acid, ℓ-glutamic acid hydrochloride, tartaric acid, and mixtures thereof, at a level such that the mole ratio of amidoamine to acid is from about 1:0.3 to about 1:1:

Preferably, in Conditioning composition B, polyoxyalkylene derivative further comprises polyoxyethylene/polyoxypropylene copolymer, more preferably polyoxyethylene/polyoxypropylene block copolymer. Preferably Conditioning composition B further contain an inert carrier, more preferably, polyethylene glycol.

### ADDITIONAL COMPONENTS

The hair conditioning composition of the present invention may include other additional components, which may be selected by the artisan according to the desired characteristics of the final product and which are suitable for rendering the composition more cosmetically or aesthetically acceptable or to provide them with additional usage benefits.

### Non-heat generating particles

Non-heat generating particles can be formulated into the present compositions. The particle can be included in the compositions at a level by weight of, preferably from about 0.01 % to about 10%, more preferably from about 0.1% to about 5%, still more preferably from about 0.1 % to about 2%. The particles useful herein has an average particle size of preferably from about 25µm to about 1500µm, more preferably from about 50µm to about 1000µm, still more preferably from about 50µm to about 500µm. Both organic and inorganic particles can be used herein. Preferred particles useful herein include organic particles such as cellulose particles, and inorganic particles such as mica, silica, mud, clay, and mixtures thereof. More preferred is silica. Some non-heat generating particles described herein can also be used as the "VISCOSITY MODIFYING AGENT" described above. Preferred particles useful herein can be those having a breakability such that the particles are breakable when the particles contained in the compositions are spread on the hands and/or on the hair. Commercially available particles useful herein include: silica having tradename Neosil series such as Neosil CBT 60 available from Crosfield.

### Cationic conditioning agent

The hair conditioning composition of the present invention may contain a cationic conditioning agent. The cationic conditioning agent can be included in the composition at a level by weight of, preferably from about 0.1 % to about 10%, more preferably from about 0.25% to about 8%, still more preferably from about 0.5% to about 3%.

The cationic conditioning agent is selected from the group consisting of cationic surfactants, cationic polymers, and mixtures thereof.

Nonlimiting examples of preferred cationic surfactants include: behenyl trimethyl ammonium chloride available, for example, with tradename INCROQUAT TMC-80 from Croda and ECONOL TM22 from Sanyo Kasei; cetyl trimethyl ammonium chloride available, for example, with tradename CA-2350 from Nikko Chemicals, hydrogenated tallow alkyl trimethyl ammonium chloride, dialkyl (14-18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride; di (behenyl/arachidyl) dimethyl ammonium chloride, dibehenyl dimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, and. N-(stearoyl colamino formyl methy) pyridinium chloride.

Suitable cationic hair conditioning polymers include, for example: copolymers of 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquatemium-16), such as those commercially available from BASF Wyandotte Corp. (Parsippany, NJ, USA) under the LUVIQUAT tradename (e.g., LUVIQUAT FC 370); copolymers of 1-vinyl-2-pyrrolidone and dimethylaminoethyl methacrylate (referred to in the industry by CTFA as Polyquatemium-11) such as those commercially available from Gaf Corporation (Wayne, NJ, USA) under the GAFQUAT tradename (e.g., GAFQUAT 755N); cationic diallyl quaternary ammonium-containing polymers, including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; and mineral acid salts of amino-alkyl esters of homo- and co-polymers of unsaturated carboxylic acids having from 3 to 5 carbon atoms, as described in U.S. Patent 4,009,256.

Other cationic polymers that can be used include polysaccharide polymers, such as cationic cellulose derivatives and cationic starch derivatives.

Cationic cellulose is available from Amerchol Corp. (Edison, NJ, USA) in their Polymer JR® and LR® series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200®.

Other cationic polymers that can be used include cationic guar gum derivatives, such as guar hydroxypropyltrimonium chloride (commercially available from Celanese Corp. in their Jaguar R series). Other materials include quaternary nitrogen-containing cellulose ethers (e.g., as described in U.S. Patent 3,962,418 and copolymers of etherified cellulose and starch (e.g., as described in U.S. Patent 3,958,581.

### Low melting point oil

The hair conditioning composition of the present invention may contain a low melting point oil, which has a melting point of less than 25°C. The low melting point oil can be included in the composition at a level by weight of, preferably from about 0.1 % to about 10%, more preferably from about 0.25% to about 6%. The low melting point oil can be used as the "INERT CARRIER" described above.

The low melting point oil useful herein is selected from the group consisting of hydrocarbon having from 10 to about 40 carbon atoms, unsaturated fatty alcohols having from about 10 to about 30 carbon atoms, unsaturated fatty acids having from about 10 to about 30 carbon atoms, fatty acid derivatives, fatty alcohol derivatives, ester oils, poly α-olefin oils, and mixtures thereof.

Fatty alcohols useful herein include those having from about 10 to about 30 carbon atoms, preferably from about 12 to about 22 carbon atoms, and more preferably from about 16 to about 22 carbon atoms. These fatty alcohols are unsaturated and can be straight or branched chain alcohols. Suitable fatty alcohols include, for example, oleyl alcohol, isostearyl alcohol, tridecylalcohol, decyl tetradecyl alcohol, and octyl dodecyl alcohol. These alcohols are available, for example, from Shinnihon Rika.

Low melting point oils useful herein include pentaerythritol ester oils, trimethylol ester oils, poly α-olefin oils, citrate ester oils, glyceryl ester oils, and mixtures thereof, and the ester oil useful herein is water-insoluble. As used herein, the term "water-insoluble" means the compound is substantially not soluble in water at 25°C; when the compound is mixed with water at a concentration by weight of above 1.0%, preferably at above 0.5%, the compound is temporarily dispersed to form an unstable colloid in water, then is quickly separated from water into two phases.

Particularly useful pentaerythritol ester oils and trimethylol ester oils herein include pentaerythritol tetraisostearate, pentaerythritol tetraoleate, trimethylolpropane triisostearate, trimethylolpropane trioleate, and mixtures thereof. Such compounds are available from Kokyo Alcohol with tradenames KAKPTI, KAKTTI, and Shin-nihon Rika with tradenames PTO, ENUJERUBU TP3SO.

Particularly useful poly α-olefin oils herein include polydecenes with tradenames PURESYN 6 having a number average molecular weight of about 500 and PURESYN 100 having a number average molecular weight of about 3000 and PURESYN 300 having a number average molecular weight of about 6000 available from Mobil Chemical Co.

Particularly useful citrate ester oils herein include triisocetyl citrate with tradename CITMOL 316 available from Bernel, triisostearyl citrate with tradename PELEMOL TISC available from Phoenix, and trioctyldodecyl citrate with tradename CITMOL 320 available from Bernel.

Particularly useful glyceryl ester oils herein include triisostearin with tradename SUN ESPOL G-318 available from Taiyo Kagaku, triolein with tradename CITHROL GTO available from Croda Surfactants Ltd., trilinolein with tradename EFADERMA-F available from Vevy, or tradename EFA-GLYCERIDES from Brooks.

### Silicone compound

The hair conditioning composition of the present invention may contain silicone compound. The silicone compound can be included in the composition at a level by weight of, preferably from about 0.1 % to about 10%, more preferably from about 0.25% to about 8%, still more preferably from about 0.5% to about 3%.

The silicone compounds hereof can include volatile soluble or insoluble, or nonvolatile soluble or insoluble silicone conditioning agents. By the term "soluble", what is meant is that the silicone compound is miscible with the carrier of the composition so as to form part of the same phase. By term "insoluble", what is meant is that the silicone forms a separate, discontinuous phase from the carrier, such as in the form of an emulsion or a suspension of droplets of the silicone. The silicone compounds herein may be made by conventional polymerization, or emulsion polymerization.

The silicone compounds for use herein will preferably have a viscosity of from about 1,000 to about 2,000,000 centistokes at 25°C, more preferably from about 10,000 to about 1,800,000, and even more preferably from about 25,000 to about 1,500,000. The viscosity can be measured by means of a glass capillary viscometer as set forth in Dow Coming Corporate Test Method CTM0004, July 20, 1970, which is incorporated by reference herein in its entirety. Silicone compound of high molecular weight may be made by emulsion polymerization.

Preferred silicone compounds are polydimethylsiloxane, polydiethylsiloxane, and polymethylphenylsiloxane. Polydimethylsiloxane, which is also known as dimethicone, is especially preferred. The polyalkylsiloxanes that can be used include, for example, polydimethylsiloxanes. These silicone compounds are available, for example, from the General Electric Company in their ViscasilR and SF 96 series, and from Dow Coming in their Dow Coming 200 series. Polymethylphenylsiloxanes, for example, from the General Electric Company as SF 1075 methyl phenyl fluid or from Dow Coming as 556 Cosmetic Grade Fluid, are useful herein.

Another silicone compound that can be especially useful is a silicone gum. The term "silicone gum", as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. Silicone gums are described by Petrarch, and others including U.S. Patent No. 4,152,416, to Spitzer et al., issued May 1, 1979 and Noll, Walter, Chemistry and Technology of Silicones, New York: Academic Press 1968. Also describing silicone gums are General Electric Silicone Rubber Product Data Sheets SE 30, SE 33, SE 54 and SE 76. All of these described references are incorporated herein by reference in their entirety. The "silicone gums" will typically have a mass molecular weight in excess of about 200,000, generally between about 200,000 and about 1,000,000. Specific examples include polydimethylsiloxane, poly(dimethylsiloxane methylvinylsiloxane) copolymer, poly(dimethylsiloxane diphenylsiloxane methylvinyisiloxane) copolymer and mixtures thereof.

Polyalkyleneoxide-modified siloxanes useful herein include, for example, polypropylene oxide modified and polyethylene oxide modified polydimethylsiloxane. The ethylene oxide and polypropylene oxide level should be sufficiently low so as not to interfere with the dispersibility characteristics of the silicone. These materials are also known as dimethicone copolyols.

Amino-substituted siloxanes known as "amodimethicone" are also useful herein. Especially preferred amino-substituted siloxane is a polymer known as "trimethylsilylamodimethicone". Another preferred amino-substituted siloxanes are those having the tradename "UCAR SILICONE ALE 56" available from Union Carbide.

### Polypropylene glycol

The hair conditioning composition of the present invention may contain a polypropylene glycol as a conditioning agent. The polypropylene glycol can be included in the composition at a level by weight of, preferably from about 0.1 % to about 10%, more preferably from about 0.25% to about 6%. Some polypropylene glycol described herein can also be used as the "VISCOSITY MODIFYING AGENTS" described above.

The polypropylene glycol useful herein may has a weight average molecular weight of preferably from about 200 g/mol to about 100,000 g/mol, more preferably from about 1,000 g/mol to about 60,000 g/mol. Without intending to be limited by theory, it is believed that the polypropylene glycol herein deposits onto, or is absorbed into hair to act as a moisturizer buffer, and/or provides one or more other desirable hair conditioning benefits. As used herein, the term "polypropylene glycol" includes single-polypropylene glycol-chain segment polymers, and multi-polypropylene glycol-chain segment polymers. The general structure of branched polymers such as the multi-polypropylene glycol-chain segment polymers herein are described, for example, in "Principles of Polymerization," pp. 17-19, G. Odian, (John Wiley & Sons, Inc., 3rd ed., 1991).

The polypropylene glycol useful herein may be either water-soluble, water-insoluble, or may have a limited solubility in water, depending upon the degree of polymerization and whether other moieties are attached thereto. The desired solubility of the polypropylene glycol in water may depend in large part upon the form (e.g., leave-on, or rinse-off form) of the hair-care composition. For example, for a rinse-off hair care composition, it is preferred that the polypropylene glycol herein has a solubility in water at 25 °C of less than about 1 g/100 g water, more preferably a solubility in water of less than about 0.5 g/100 g water, and even more preferably a solubility in water of less than about 0.1 g/100 g water.

Preferably the polypropylene glycol is selected from the group consisting of a single-polypropylene glycol-chain segment polymer, a multi-polypropylene glycol-chain segment polymer, and mixtures thereof, more preferably selected from the group consisting of a single-polypropylene glycol-chain segment polymer of Formula I, below, a multi-polypropylene glycol-chain segment polymer of Formula II, below, and mixtures thereof.

Accordingly, a highly preferred single-polypropylene glycol-chain segment polymer has the formula:

HO-(C₃H₆O)ₐH (III),

wherein a is a value from about 4 to about 400, preferably from about 20 to about 100, and more preferably from about 20 to about 40.

The single-polypropylene glycol-chain segment polymer useful herein is typically inexpensive, and is readily available from, for example, Sanyo Kasei (Osaka, Japan), Dow Chemicals (Midland, Michigan, USA), Calgon Chemical, Inc. (Skokie, Illinois, USA), Arco Chemical Co. (Newton Square Pennsylvania, USA), Witco Chemicals Corp. (Greenwich, Connecticut, USA), and PPG Specialty Chemicals (Gumee, Illinois, USA).

A highly preferred multi-polypropylene glycol-chain segment polymer has the formula: 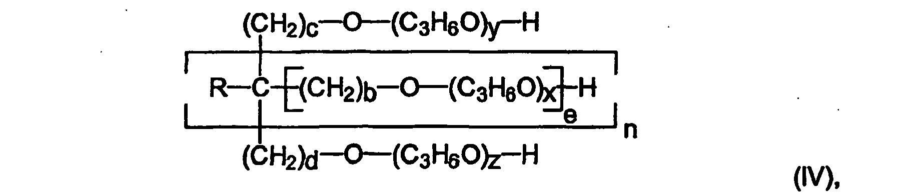 wherein n is a value from about 0 to about 10, preferably from about 0 to about 7, and more preferably from about 1 to about 4. In Formula IV, each R" is independently selected from the group consisting of H, and C₁-C₃₀ alkyl, and preferably each R" is independently selected from the group consisting of H, and C₁-C₄ alkyl. In Formula IV, each b is independently a value from about 0 to about 2, preferably from about 0 to about 1, and more preferably b = 0. Similarly, c and d are independently a value from about 0 to about 2, preferably from about 0 to about 1. However, the total of b + c + d is at least about 2, preferably the total of b + c + d is from about 2 to about 3. Each e is independently a value of 0. or 1, if n is from about 1 to about 4, then e is preferably equal to 1. Also in Formula IV, x, y, and z is independently a value of from about 1 to about 120, preferably from about 7 to about 100, and more preferably from about 7 to about 100, where x + y + z is greater than about 20.

Examples of the multi-polypropylene glycol-chain segment polymer of Formula IV which is especially useful herein includes polyoxypropylene glyceryl ether (n = 1, R' = H, b = 0, c and d = 1, e = 1, and x, y, and z independently indicate the degree of polymerization of their respective polypropylene glycol-chain segments; available as New Pol GP-4000, from Sanyo Kasei, Osaka, Japan), polypropylene trimethylol propane (n =1, R' = C₂H₅, b = 1, c and d = 1, e =1, and x, y, and z independently indicate the degree of polymerization of their respective polypropylene glycol-chain segments), polyoxypropylene sorbitol (n = 4, each R' = H, b = 0, c and d = 1, each e =1, and y, z, and each x independently indicate the degree of polymerization of their respective polypropylene glycol-chain segments; available as New Pol SP-4000, from Sanyo Kasei, Osaka, Japan), and PPG-10 butanediol (n = 0, c and d = 2, and y + z = 10; available as Probutyl DB-10, from Croda, Inc., of Parsippany, New Jersey, U.S.A.).

In a preferred embodiment, one or more of the propylene repeating groups in the polypropylene glycol is an isopropyl oxide repeating group. More preferably one or more of the propylene oxide repeating groups of the polypropylene glycol of Formula III and/or the polypropylene glycol of Formula IV is an isopropyl oxide repeating group. Even more preferably, substantially all of the propylene oxide repeating groups of the polypropylene glycol of Formula III and/or the polypropylene glycol of Formula IV are isopropyl oxide repeating groups. Accordingly, a highly preferred single-polypropylene glycol-chain segment polymer has the formula: 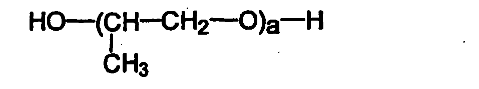 wherein a is defined as described above for Formula III. Similarly, a highly preferred multi-polypropylene glycol-chain segment polymer has the formula: 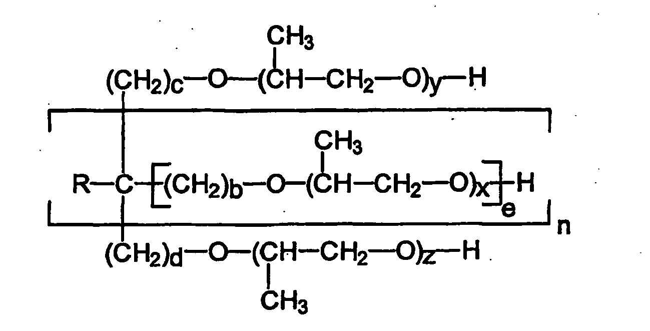 wherein n, R", b, c, d, e, x, y, and z are defined as above, for Formula- IV. It is recognized that the isopropyl oxide repeating groups may also correspond either alone, or in combination with the above depicted, to:

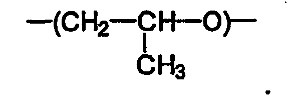

The polypropylene glycol useful herein is readily available from, for example, Sanyo Kasei (Osaka, Japan) as New pol PP-2000, New pol PP-4000, New pol GP-4000, and New pol SP-4000, from Dow Chemicals (Midland, Michigan, USA), from Calgon Chemical, Inc. (Skokie, Illinois, USA), from Arco Chemical Co. (Newton Square Pennsylvania, USA), from Witco Chemicals Corp. (Greenwich, Connecticut, USA), and from PPG Specialty Chemicals (Gumee, Illinois, USA). High molecular weight polyethylene glycol

The hair conditioning composition of present invention may contain a high molecular weight polyethylene glycol as a conditioning agent The high molecular weight polyethylene glycol can also be used as the "VISCOSITY MODIFYING AGENT" described above. The polyethylene glycols useful herein are those having the formula:

H(OCH₂CH₂)ₙ -OH

wherein n has an average value of from 2,000 to 14,000, preferably from about 5,000 to about 9,000, more preferably from about 6,000 to about 8,000.

The polyethylene glycol can be included in the composition at a level by weight of, preferably from about 0.1 % to about 10%, more preferably from about 0.25% to about 6%.

The polyethylene glycol described above is also known as a polyethylene oxide, and polyoxyethylene. Polyethylene glycols useful herein that are especially preferred are PEG-2M wherein n has an average value of about 2,000 (PEG-2M is also known as Polyox WSR® N-10 from Union Carbide and as PEG-2,000); PEG-5M wherein n has an average value of about 5,000 (PEG-5M is also known as Polyox WSR® N-35 and as Polyox WSR® N-80, both from Union Carbide and as PEG-5,000 and Polyethylene Glycol 300,000); PEG-7M wherein n has an average value of about 7,000 (PEG-7M is also known as Polyox WSR® N-750 from Union Carbide); PEG-9M wherein n has an average value of about 9,000 (PEG-9M is also known as Polyox WSR® N-3333 from Union Carbide); and PEG-14M wherein n has an average value of about 14,000 (PEG-14M is also known as Polyox WSR® N-3000 from Union Carbide).

### Other additional components

A wide variety of other additional components can be formulated into the present compositions. These include: other conditioning agents such as hydrolyzed collagen with tradename Peptein 2000 available from Hormel, vitamin E with tradename Emix-d available from Eisai, panthenol available from Roche, panthenyl ethyl ether available from Roche, a mixture of Polysorbate 60 and Cetearyl Alcohol with tradename Polawax NF available from Croda Chemicals, glycerylmonostearate available from Stepan Chemicals, hydroxyethyl cellulose available from Aqualon, 3-pyridinecarboxy acid amide (niacinamide), hydrolysed keratin, proteins, plant extracts, and nutrients; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents such as the thioglycolates; perfumes; and sequestering agents, such as disodium ethylenediamine tetra-acetate; ultraviolet and infrared screening and absorbing agents such as octyl salicylate, antidandruff agents such as zinc pyridinethione, and salicylic acid; and optical brighteners, for example polystyrylstilbenes, triazinstilbenes, hydroxycoumarins, aminocoumarins, triazoles, pyrazolines, oxazoles, pyrenes, porphyrins, imidazoles, and mixtures thereof.

Other additional components generally are used individually at levels of from about 0.001 % to about 10%, preferably up to about 5% by weight of the composition.

### EXAMPLES

The following examples further describe and demonstrate embodiments within the scope of the present invention. Ingredients are identified by chemical or CTFA name, or otherwise defined below.

### Hair Conditioning Compositions

| Components | Ex. 1 | Ex. 2 | Ex. 3 |
|---|---|---|---|
| Anhydrous magnesium sulfate (MgSO₄) | 35 | 25 | 45 |
| Polyethylene/polypropylene block copolymer *1 | 3.0 | 1.0 | 5.0 |
| Cetyl Alcohol *4 | 1.0 | 2.0 | 0.25 |
| Stearyl Alcohol *5 | 1.4 | 2.8 | 0.45 |
| Stearamidopropyl Dimethylamine *6 | 0.8 | 1.6 | 0.2 |
| ℓ-Glutamic acid *7 | 0.256 | 0.512 | 0.064 |
| Hydroxyethylcellulose *8 | - | 2.0 | 0.5 |
| Perfume | 0.3 | 0.3 | 0.3 |
| Benzyl alcohol | - | - | 0.4 |
| EDTA | - | - | 0.1 |
| Kathon CG *9 | - | - | 0.0005 |
| Sodium Chloride | - | - | 0.01 |
| 3-pyridinecarboxy acid amide | 0.05 | 0.05 | 0.05 |
| dl-Alpha tocopherol acetate | 0.05 | 0.05 | 0.05 |
| Hydrolyzed collagen *10 | 0.01 | 0.01 | 0.01 |
| Panthenol *11 | 0.05 | 0.05 | 0.05 |
| Panthenyl Ethyl Ether *12 | 0.05 | 0.05 | 0.05 |
| Octyl methoxycinnamate | 0.09 | 0.09 | 0.09 |
| Benzophenone-3 | 0.09 | 0.09 | 0.09 |
| Polyethylene glycol *13 | q.s. to 100% | q.s. to 100% | q.s. to 100% |

### Hair Conditioning Compositions

| Components | Ex.4 |
|---|---|
| Anhydrous magnesium sulfate (MgSO₄) | - |
| Anhydrous calcium chloride (CaCl₂) | 35 |
| Polyethylene/polypropylene block copolymer*1 | 3.0 |
| PEG modified glyceride *2 | - |
| Cetyl Alcohol *4 | 1.0 |
| Stearyl Alcohol *5 | 1.4 |
| Stearamidopropyl Dimethylamine *6 | 0.8 |
| ℓ-Glutamine acid *7 | 0.256 |
| Hydroxyethylcellulose *8 | 0.5 |
| Perfume | 0.3 |
| Benzyl alcohol | 0.4 |
| EDTA | 0.1 |
| Kathon CG *9 | 0.0005 |
| Sodium Chloride | 0.01 |
| 3-pyridinecarboxy acid amide | 0.05 |
| dl-Alpha tocopherol acetate | 0.05 |
| Hydrolyzed collagen *10 | 0.01 |
| Panthenol *11 | 0.05 |
| Panthenyl Ethyl Ether *12 | 0.05 |
| Octyl methoxycinnamate | 0.09 |
| Benzophenone-3 | 0.09 |
| Polyethylene glycol *13 | - |
| Glycerin | q.s. to 100% |
| Pentaerythritol Tetraisostearate *14 | - |

### Hair Conditioning Compositions

| Components | Ex. 5 |
|---|---|
| Anhydrous magnesium sulfate (MgSO₄) | - |
| Anhydrous calcium chloride (CaCl₂) | - |
| Anhydrous magnesium chloride (MgCl₂) | 35 |
| Polyethylene/polypropylene block copolymer *1 | 3.0 |
| PEG modified glyceride *2 | - |
| PEG-60 hydrogenated caster oil *3 | |
| Cetyl Alcohol *4 | 1.0 |
| Stearyl Alcohol *5 | 1.4 |
| Stearamidopropyl Dimethylamine *6 | 0.8 |
| ℓ-Glutamic acid *7 | - |
| Hydroxyethylcellulose *8 | 0.1 |
| Silicone blend *15 | 1.0 |
| Polypropylene Glycol *16 | |
| Ditallow dimethyl ammonium chloride *17 | 0.3 |
| PEG-2M*18 | 0.2 |
| Polysorbate 60 *19 | 0.2 |
| Cetearyl alcohol *19 | 0.2 |
| Benzyl alcohol | 0.2 |
| Glyceryl monostearate *20 | 0.2 |
| Oleyl alcohol *21 | 0.2 |
| Perfume | 0.3 |
| Benzyl alcohol | 0.4 |
| EDTA | 0.1 |
| Kathon CG *9 | 0.0005 |
| Sodium Chloride | 0.01 |
| 3-pyridinecarboxy acid amide | 0.05 |
| dI-Alpha tocopherol acetate | 0.05 |
| Hydrolyzed collagen *10 | 0.01 |
| Panthenol *11 | 0.05 |
| Panthenyl Ethyl Ether *12 | 0.05 |
| Octyl methoxycinnamate | 0.09 |
| Benzophenone-3 | 0.09 |
| Polyethylene glycol *13 | q.s. to 100% |
| Pentaerythritol Tetraisostearate *14 | - |

### Hair Conditioning Compositions

| Components | Ex. 6 | Ex. 7 | Ex. 8 |
|---|---|---|---|
| Anhydrous magnesium sulfate (MgSO₄) | 35 | 25 | 45 |
| Polyethylene/polypropylene block copolymer *1 | 3.0 | 1.0 | 5.0 |
| Cetyl Alcohol *4 | 1.0 | 2.0 | 0.25 |
| Stearyl Alcohol *5 | 1.4 | 2.8 | 0.45 |
| Stearamidopropyl Dimethylamine *6 | 0.8 | 1.6 | 0.2 |
| ℓ-Glutamic acid *7 | 0.256 | 0.512 | 0.064 |
| Hydroxyethylcellulose *8 | 0.5 | 2.0 | 0.5 |
| Perfume | 0.3 | 0.3 | 0.3 |
| Benzyl alcohol | - | - | 0.4 |
| EDTA | - | - | 0.1 |
| Kathon CG *9 | - | - | 0.0005 |
| Sodium Chloride | - | - | 0.01 |
| 3-pyridinecarboxy acid amide | 0.05 | 0.05 | 0.05 |
| dl-Alpha tocopherol acetate | 0.05 | 0.05 | 0.05 |
| Hydrolyzed collagen *10 | 0.01 | 0.01 | 0.01 |
| Panthenol *11 | 0.05 | 0.05 | 0.05 |
| Panthenyl Ethyl Ether *12 | 0.05 | 0.05 | 0.05 |
| Octyl methoxycinnamate | 0.09 | 0.09 | 0.09 |
| Benzophenone-3 | 0.09 | 0.09 | 0.09 |
| Polyethylene glycol *13 | q.s. to 100% | q.s. to 100% | - |
| Polyethylene glyceryl ester *22 | 20 | 30 | q.s. to 100% |

| | | | |
|---|---|---|---|
| Definitions of Components ^{*}1 Polyethylene/polypropylene block copolymer: Newpol PE-108 available from Sanyo Chemical. ^{*}2 PEG modified glyceride: Tagat TO available from Goldschmidt Chemical Corporation. ^{*}3 PEG-60 hydrogenated caster oil: Cremophor RH60 available from BASF. ^{*}4 Cetyl Alcohol: Konol series available from Shin Nihon Rika. ^{*}5 Stearyl Alcohol: Konol series available from Shin Nihon Rika. ^{*}6 Stearamidopropyl Dimethylamine: SAPDMA available from Inolex. ^{*}7 ℓ-Glutamic acid: ℓ-Glutamic acid (cosmetic grade) available from Ajinomoto. ^{*}8 Hydroxyethylcellulose: Natrosol 250 MBR available from Hercules. ^{*}9 Kathon CG: Methylchlorosothiazolinone and Methylisothiazolinone available from Rohm & Haas. ^{*}10 Hydrolyzed collagen: Peptein 2000 available from Hormel. ^{*}11 Panthenol: available from Roche. ^{*}12 Panthenyl Ethyl Ether: available from Roche. ^{*}13 Polyethylene glycol: Carbowax PEG-200 available from Union Carbide. ^{*}14 Pentaerythritol Tetraisostearate: KAK PTI obtained by Kokyu alcohol. ^{*}15 Silicone Blend: SE 76 available from General Electric ^{*}16 Polypropylene Glycol: PP2000 available from Sanyo Kasei ^{*}17 Ditallow dimethyl ammonium chloride: Available from Witco Chemicals. ^{*}18 PEG-2M: Polyox obtained by Union Carbide. ^{*}19 Polysorbate 60, Cetearyl Alcohol: mixture sold as Polawax NF obtained by Croda Chemicals. ^{*}20 Glycerylmonostearate: Available from Stepan Chemicals. ^{*}21 Oleyl alcohol: Available from New Japan Chemical. ^{*}22 Polyethylene glyceryl ester. Tagat TO ® available from Goldschmidt Chemical Corporation | | | |

### Method of Preparation

The hair conditioning compositions of Examples 1 through 12 as shown above can be prepared by any conventional method well known in the art. They are suitably made as follows: When included in the composition, polymeric materials such as hydroxyethylcellulose can be dispersed in the inert carrier such as polyethylene glycol (PEG-200) at room temperature to make a polymer solution, and heated up to above 70°C. Such polymeric materials can be also dispersed in polyoxyalkylene derivatives such as polyoxyethylene glyceryl ether, in case the polyoxyalkylene derivatives are included at 10% or more in the final composition. Other polyoxyalkylene derivatives such as polyoxyethylene/polyoxyalkylene copolymer, and when present, amidoamines and acids, cationic surfactants, high melting point fatty compounds, and ester oils of low melting point oils are added in the solution with agitation. Then, inorganic heat generating agents such as magnesium sulfate, calcium chloride, and magnesium chloride are also added in the solution with agitation. The mixture thus obtained is cooled down to about 30°C, and the remaining components such as silicone compound are added with agitation.

### Method of Use

The hair conditioning compositions of Examples 1 through 12 as shown above can be mixed with water and applied to the hair and/or skin by any conventional method well known in the art. For example, the anhydrous compositions can be applied to hair and/or skin after mixing with water on hands and/or in a certain vessel. The anhydrous compositions can be applied to wet hair and/or wet skin to mix with water remaining on the hair and/or skin. The anhydrous compositions can be applied to wet and/or dry hair and/or skin to mix with water when rinsed-off. The hair conditioning compositions of Examples 1 through 12 as shown above are preferably applied to wet hair to mix with water remaining on the hair.

The embodiments disclosed herein have many advantages. For example, anhydrous cosmetic compositions of the present invention, can provide enhanced efficacy, i.e., can provide improved benefits, while reducing gritty feel to the skin and/or hair. For example, warming hair conditioning compositions can provide improved hair conditioning benefits such as moisturized feel, softness, and static control to the hair, due to improved penetration of ingredients, while reducing gritty feel to the hair, hair scalp, and/or hands.

## Claims

1. An anhydrous cosmetic composition comprising:
(a) an inorganic heat generating agent which generates a heat by mixing with water; and
(b) a polyoxyalkylene derivative
wherein the inorganic heat generating agent is an anhydrous inorganic salt selected from the group consisting of sodium sulfate, calcium sulfate, magnesium sulfate, aluminum sulfate, calcium chloride, magnesium chloride, calcium oxide, and mixtures thereof, and
wherein the polyoxyalkylene derivative is selected from the group consisting of poiyoxyethylene/polyoxypropylene copolymer, polyoxyethylene glyceryl ester, and mixtures thereof, and
wherein the polyoxyethylene/polyoxypropylene copolymer is included in said composition at a level by weight of, from 0.1% to 10%, preferably from 0.5% to 10%, more preferably from 1 % to 5%, and
wherein the polyoxyethylene glyceryl ester is included in said composition at a level by weight of, from 10% to 90%, preferably from 15% to 85%, more preferably from 20% to 80%.

2. The anhydrous cosmetic composition according to Claim 1, further comprising an inert carrier.

3. The anhydrous cosmetic composition according to Claim 1 and 2, wherein the inorganic heat generating agent has an average diameter of from about 0.01 µm to about 40µm.

4. The anhydrous cosmetic composition according to any of the preceding Claims, wherein the polyoxyethylene/pofyoxypropylene copolymer is a polyoxyethylene/polyoxypropylene block copolymer.

5. The anhydrous cosmetic composition according to Claim 2, 3 and 4, wherein the inert carrier is selected from the group consisting of polyethylene glycol, polypropylene glycol, glycerin, liquid paraffin, mineral oil, vegetable oil, pentaerythritol tetraisostearate, and mixtures thereof.

6. The anhydrous cosmetic composition according to Claim 5, wherein the inert carrier is polyethylene glycol.

7. The anhydrous cosmetic composition according to any of the preceding claims, further comprising a reaction control agent.

8. The anhydrous cosmetic composition according to Claim 7, wherein the reaction control agent is selected from the group consisting of cellulose derivatives, modified cellulose polymers, and mixtures thereof.

9. The anhydrous cosmetic composition according to any of the preceding claims, further comprising a heat reserving material.

10. The anhydrous cosmetic composition according to Claim 9, wherein the heat reserving material is a phase-changing material having a melting point of from about 25°C to about 80°C.

11. The anhydrous cosmetic composition according to Claim 10, wherein the heat reserving material is selected from the group consisting of fatty alcohols, fatty acids, and mixtures thereof.

12. The anhydrous cosmetic composition according to any of the preceding claims, which warms to a temperature of from about 25°C to about 80°C by mixing with water.

13. The anhydrous cosmetic composition according to any of the preceding claims, which is an anhydrous hair care composition selected from the group consisting of an anhydrous hair shampoo composition, an anhydrous hair styling composition, an anhydrous hair conditioning composition, an anhydrous hair colour composition, an anhydrous hair growth composition, and mixtures thereof.

14. The anhydrous cosmetic composition according to Claim 13, which is an anhydrous hair conditioning composition.

15. The anhydrous cosmetic composition according to Claim 14, wherein the anhydrous hair conditioning composition further comprises a high melting point fatty compound.

16. The anhydrous cosmetic composition according to Claim 14, wherein the anhydrous hair conditioning composition further comprises an amidoamine having the following general formula:
R¹ CONH (CH₂)ₘ N (R²)₂
wherein R¹ is a residue of C₁₁ to C₂₄ fatty acids, R² is a C₁ to C₄ alkyl, and m is an integer from 1 to 4; and an acid selected from the group consisting of ℓ-glutamic acid, lactic acid, hydrochloric acid, malic acid, succinic acid, acetic acid, fumaric acid, ℓ-glutamic acid hydrochloride, tartaric acid, and mixtures thereof, at a level such that the mole ratio of amidoamine to acid is from about 1:0.3 to about 1:1.

17. The anhydrous cosmetic composition according to Claim 14, wherein the anhydrous hair conditioning composition comprises by weight:
(a) from 5% to 60% of the inorganic heat generating agent which generates a heat by mixing with water;
(b) from 0.1% to 10% of the polyoxyalkylene derivative comprising a polyoxyethylene/polyoxypropylene copolymer;
(c) from 0.1 % to 30% of a high melting point fatty compound;
(d) from 0.05% to 10% of an amidoamine having the following general formula:
R¹ CONH (CH₂)ₘ N (R²)₂
wherein R¹ is a residue of C₁₁ to C₂₄ fatty acids, R² is a C₁ to C₄ alkyl, and m is an integer from 1 to 4;
(e) an acid selected from the group consisting of ℓ-glutamic acid, lactic acid, hydrochloric acid, malic acid, succinic acid, acetic acid, fumaric acid, ℓ-glutamic acid hydrochloride, tartaric acid, and mixtures thereof, at a level such that the mole ratio of amidoamine to acid is from about 1:0.3 to about 1:1; and
(f) an inert carrier.

18. The anhydrous cosmetic composition according to Claim 14, wherein the anhydrous hair conditioning composition comprising by weight:
(a) from 5% to 60% of the inorganic heat generating agent which generates a heat by mixing with water;
(b) from 10% to 90% of the polyoxyalkylene derivative comprising a polyoxyethylene glyceryl ester;
(c) from 0.1 % to 30% of a high melting point fatty compound;
(d) from 0.05% to 10% of an amidoamine having the following general formula:
R¹ CONH (CH₂)ₘ N (R²)₂
wherein R¹ is a residue of C₁₁ to C₂₄ fatty acids, R² is a C₁ to C₄ alkyl, and m is an integer from 1 to 4;
(e) an acid selected from the group consisting of ℓ-glutamic acid, lactic acid, hydrochloric acid, malic acid, succinic acid, acetic acid, fumaric acid, ℓ-glutamic acid hydrochloride, tartaric acid, and mixtures thereof, at a level such that the mole ratio of amidoamine to acid is from about 1:0.3 to about 1:1.

19. A method of using the hair conditioning composition according to Claim 14,
wherein the composition is applied to wet hair to mix with water remaining on the hair.

## Patentansprüche

1. Wasserfreie Kosmetikzusammensetzung, umfassend:
(a) ein anorganisches, Wärme erzeugendes Mittel, das durch Vermischen mit Wasser Wärme erzeugt; und
(b) ein Polyoxyalkylenderivat.
wobei das anorganische, Wärme erzeugende Mittel ein wasserfreies anorganisches Salz ist, ausgewählt aus der Gruppe, bestehend aus Natriumsulfat, Calciumsulfat, Magnesiumsulfat, Aluminiumsulfat, Calciumchlorid, Magnesiumchlorid, Calciumoxid und Mischungen davon, und
wobei das Polyoxyalkylenderivat ausgewählt ist aus der Gruppe, bestehend aus Polyoxyethylen-/Polyoxypropylen-Copolymer, Polyoxyethylenglycerylester und Mischungen davon, und
wobei das Polyoxyethylen-/Polyoxypropylen-Copolymer in der Zusammensetzung in einer Konzentration von 0,1 Gew.-% bis 10 Gew.-%, vorzugsweise von 0,5 Gew.-% bis 10 Gew.-%, mehr bevorzugt von 1 Gew.-% bis 5 Gew.-% enthalten ist, und
wobei der Polyoxyethylenglycerylester in der Zusammensetzung in einer Konzentration von 10 Gew.-% bis 90 Gew.-%, vorzugsweise von 15 Gew.-% bis 85 Gew.-%, mehr bevorzugt von 20 Gew.-% bis 80 Gew.-% enthalten ist.

2. Wasserfreie Kosmetikzusammensetzung nach Anspruch 1, die ferner einen inerten Träger umfasst.

3. Wasserfreie Kosmetikzusammensetzung nach Anspruch 1 und 2, wobei das anorganische, Wärme erzeugende Mittel einen durchschnittlichen Durchmesser von ungefähr 0,01 µm bis ungefähr 40 µm aufweist.

4. Wasserfreie Kosmetikzusammensetzung nach einem der vorstehenden Ansprüche, wobei das Polyoxyethylen-/Polyoxypropylen-Copolymer ein Polyoxyethylen-/Polyoxypropylen-Blockpolymer ist.

5. Wasserfreie Kosmetikzusammensetzung nach Anspruch 2, 3 und 4, wobei der inerte Träger ausgewählt ist aus der Gruppe, bestehend aus Polyethylenglycol, Polypropylenglycol, Glycerin, flüssigem Paraffin, Mineralöl, Pflanzenöl, Pentaerythrittetraisostearat und Mischungen davon.

6. Wasserfreie Kosmetikzusammensetzung nach Anspruch 5, wobei der inerte Träger Polyethylenglycol ist.

7. Wasserfreie Kosmetikzusammensetzung nach einem der vorstehenden Ansprüche, die ferner ein Reaktionslenkungsmittel umfasst.

8. Wasserfreie Kosmetikzusammensetzung nach Anspruch 7, wobei das Reaktionslenkungsmittel ausgewählt ist aus der Gruppe, bestehend aus Cellulosederivaten, modifizierten Cellulosepolymeren und Mischungen davon.

9. Wasserfreie Kosmetikzusammensetzung nach einem der vorstehenden Ansprüche, die ferner ein Wärmespeichermaterial umfasst.

10. Wasserfreie Kosmetikzusammensetzung nach Anspruch 9, wobei das Wärmespeichermaterial ein Phasenänderungsmaterial mit einem Schmelzpunkt von ungefähr 25 °C bis ungefähr 80 °C ist.

11. Wasserfreie Kosmetikzusammensetzung nach Anspruch 10, wobei das Wärmespeichermaterial ausgewählt ist aus der Gruppe, bestehend aus Fettalkoholen, Fettsäuren und Mischungen davon.

12. Wasserfreie Kosmetikzusammensetzung nach einem der vorstehenden Ansprüche, die durch Vermischen mit Wasser auf eine Temperatur von ungefähr 25 °C bis ungefähr 80 °C erwärmt.

13. Wasserfreie Kosmetikzusammensetzung nach einem der vorstehenden Ansprüche, die eine wasserfreie Haarpflegezusammensetzung ist, ausgewählt aus der Gruppe, bestehend aus einer wasserfreien Haarwaschmittelzusammensetzung, einer wasserfreien Haarstylingzusammensetzung, einer wasserfreien Haarkonditionierungszusammensetzung, einer wasserfreien Haarfarbzusammensetzung, einer wasserfreien Haarwuchszusammensetzung und Mischungen davon.

14. Wasserfreie Kosmetikzusammensetzung nach Anspruch 13, die eine wasserfreie Haarkonditionierungszusammensetzung ist.

15. Wasserfreie Kosmetikzusammensetzung nach Anspruch 14, wobei die wasserfreie Haarkonditionierungszusammensetzung ferner eine Fettverbindung mit hohem Schmelzpunkt umfasst.

16. Wasserfreie Kosmetikzusammensetzung nach Anspruch 14, wobei die wasserfreie Haarkonditionierungszusammensetzung ferner ein Amidoamin mit der folgenden allgemeinen Formel umfasst:
R¹ CONH (CH₂)ₘ N (R²)₂
worin R¹ ein Rest von C₁₁- bis C₂₄-Fettsäuren ist, R² ein C₁-bis C₄-Alkyl ist und m eine ganze Zahl von 1 bis 4 ist; und
eine Säure, ausgewählt aus der Gruppe, bestehend aus ℓ-Glutaminsäure, Milchsäure, Salzsäure, Apfelsäure, Bernsteinsäure, Essigsäure, Fumarsäure, ℓ-Glutaminsäurehydrochlorid, Weinsäure und Mischungen davon, in einer solchen Konzentration, dass das Molverhältnis von Amidoamin zu Säure von ungefähr 1:0,3 bis ungefähr 1:1 1 ist.

17. Wasserfreie Kosmetikzusammensetzung nach Anspruch 14, wobei die wasserfreie Haarkonditionierungszusammensetzung bezogen auf das Gewicht Folgendes umfasst:
(a) von 5 % bis 60 % das anorganische, Wärme erzeugende Mittel, das durch Vermischen mit Wasser Wärme erzeugt;
(b) von 0,1 % bis 10 % das Polyoxyalkylenderivat, das ein Polyoxyethylen/Polyoxypropylen-Copolymer umfasst;
(c) von 0,1 % bis 30 % eine Fettverbindung mit hohem Schmelzpunkt;
(d) von 0,05 % bis 10 % ein Amidoamin mit der folgenden allgemeinen Formel:
R¹ CONH (CH₂)ₘ N (R²)₂
worin R¹ ein Rest von C₁₁- bis C₂₄-Fettsäuren ist, R² ein C₁-bis C₄-Alkyl ist und m eine ganze Zahl von 1 bis 4 ist;
(e) eine Säure, ausgewählt aus der Gruppe, bestehend aus ℓ-Glutaminsäure, Milchsäure, Salzsäure, Apfelsäure, Bernsteinsäure, Essigsäure, Fumarsäure, ℓ-Glutaminsäurehydrochlorid, Weinsäure und Mischungen davon, in einer solchen Konzentration, dass das Molverhältnis von Amidoamin zu Säure von ungefähr 1:0,3 bis ungefähr 1:1 ist; und
(f) einen inerten Träger.

18. Wasserfreie Kosmetikzusammensetzung nach Anspruch 14, wobei die wasserfreie Haarkonditionierungszusammensetzung bezogen auf das Gewicht Folgendes umfasst:
(a) von 5 % bis 60 % das anorganische, Wärme erzeugende Mittel, das durch Vermischen mit Wasser Wärme erzeugt;
(b) von 10 % bis 90 % das Polyoxyalkylenderivat, das einen Polyoxyethylenglycerylester umfasst;
(c) von 0,1 % bis 30 % eine Fettverbindung mit hohem Schmelzpunkt;
(d) von 0,05 % bis ungefähr 10 % ein Amidoamin mit der folgenden allgemeinen Formel:
R¹ CONH (CH₂)ₘ N (R²)₂
worin R¹ ein Rest von C₁₁- bis C₂₄-Fettsäuren ist, R² ein C₁-bis C₄-Alkyl ist und m eine ganze Zahl von 1 bis 4 ist; und
(e) eine Säure, ausgewählt aus der Gruppe, bestehend aus ℓ-Glutaminsäure, Milchsäure, Salzsäure, Apfelsäure, Bernsteinsäure, Essigsäure, Fumarsäure, ℓ-Glutaminsäurehydrochlorid, Weinsäure und Mischungen davon, in einer solchen Konzentration, dass das Molverhältnis von Amidoamin zu Säure von ungefähr 1:0,3 bis ungefähr 1:1 ist.

19. Verfahren zum Verwenden der Haarkonditionierungszusammensetzung nach Anspruch 14, wobei die Zusammensetzung auf nasses Haar aufgetragen wird, um sich mit auf dem Haar verbleibendem Wasser zu vermischen.

## Revendications

1. Composition de produit de beauté anhydre comprenant :
(a) un agent de génération de chaleur inorganique qui génère de la chaleur lorsqu'il est mélangé à de l'eau ; et
(b) un dérivé de polyoxyalkylène.
dans laquelle l'agent de génération de chaleur inorganique est un sel inorganique anhydre choisi parmi le groupe constitué de sulfate de sodium, sulfate de calcium, sulfate de magnésium, sulfate d'aluminium, chlorure de calcium, chlorure de magnésium, oxyde de calcium, et leurs mélanges, et
dans laquelle le dérivé de polyoxyalkylène est choisi parmi le groupe constitué de copolymère de polyoxyéthylène/polyoxypropylène, d'ester glycérique de polyoxyéthylène, et leurs mélanges, et
dans laquelle le copolymère de polyoxyéthylène/polyoxypropylène est inclus dans ladite composition à un niveau en poids allant de 0,1 % à 10 %, de préférence de 0,5 % à 10 %, plus préférablement de 1 % à 5 %, et
dans laquelle l'ester glycérique de polyoxyéthylène est inclus dans ladite composition à un niveau en poids allant de 10 % à 90 %, de préférence de 15 % à 85 %, plus préférablement de 20 % à 80 %.

2. Composition de produit de beauté anhydre selon la revendication 1, comprenant en outre un véhicule inerte.

3. Composition de produit de beauté anhydre selon les revendications 1 et 2, dans laquelle l'agent de génération de chaleur inorganique a un diamètre moyen allant d'environ 0,01 µm à environ 40 µm.

4. Composition de produit de beauté anhydre selon l'une quelconque des revendications précédentes, dans laquelle le copolymère de polyoxyéthylène/polyoxypropylène est un polymère séquencé de polyoxyéthylène/polyoxypropylène.

5. Composition de produit de beauté anhydre selon les revendications 2, 3 et 4, dans laquelle le véhicule inerte est choisi parmi le groupe constitué de polyéthylène glycol, polypropylène glycol, glycérine, paraffine liquide, huile minérale, huile végétale, tétraisostéarate penta-érythritol, et leurs mélanges.

6. Composition de produit de beauté anhydre selon la revendication 5, dans laquelle le véhicule inerte est un polyéthylène glycol.

7. Composition de produit de beauté anhydre selon l'une quelconque des revendications précédentes, comprenant en outre un agent de contrôle de réaction.

8. Composition de produit de beauté anhydre selon la revendication 7, dans laquelle l'agent de contrôle de réaction est choisi parmi le groupe constitué de dérivés de cellulose, polymères cellulosiques modifiés, et leurs mélanges.

9. Composition de produit de beauté anhydre selon l'une quelconque des revendications précédentes, comprenant en outre un matériau d'accumulation de chaleur.

10. Composition de produit de beauté anhydre selon la revendication 9, dans laquelle le matériau d'accumulation de chaleur est un matériau changeant de phase ayant un point de fusion allant d'environ 25 °C à environ 80 °C.

11. Composition de produit de beauté anhydre selon la revendication 10, dans laquelle le matériau d'accumulation de chaleur est choisi parmi le groupe constitué d'alcools gras, acides gras, et leurs mélanges.

12. Composition de produit de beauté anhydre selon l'une quelconque des revendications précédentes, qui chauffe pour atteindre une température allant d'environ 25 °C à environ 80 °C en la mélangeant à de l'eau.

13. Composition de produit de beauté anhydre selon l'une quelconque des revendications précédentes, qui est une composition pour soins capillaires anhydre choisie parmi le groupe constitué d'une composition de shampoing pour cheveux anhydre, une composition de coiffage anhydre, une composition de conditionnement des cheveux anhydre, une composition de colorant pour cheveux anhydre, une composition de pousse des cheveux anhydre, et leurs mélanges.

14. Composition de produit de beauté anhydre selon la revendication 13, qui est une composition de conditionnement des cheveux anhydre.

15. Composition de produit de beauté anhydre selon la revendication 14, dans laquelle la composition de conditionnement des cheveux anhydre comprend en outre un composé gras à point de fusion élevé.

16. Composition de produit de beauté anhydre selon la revendication 14, dans laquelle la composition de conditionnement des cheveux anhydre comprend en outre une amidoamine de formule générale suivante :
R¹ CONH (CH₂)ₘ N (R²)₂
dans laquelle R¹ est un résidu d'acides gras en C₁₁ à C₂₄, R² est un alkyle en C₁ à C₄, et m est un entier compris entre 1 et 4 ; et
un acide choisi parmi le groupe constitué d'acide ℓ-glutamique, acide lactique, acide chlorhydrique, acide malique, acide succinique, acide acétique, acide fumarique, chlorhydrate d'acide ℓ-glutamique, acide diacétyltartrique, et leurs mélanges, à un niveau tel que le rapport molaire de l'amidoamine sur l'acide est d'environ 1:0,3 à environ 1:1.

17. Composition de produit de beauté anhydre selon la revendication 14, dans laquelle la composition de conditionnement des cheveux anhydre comprend en poids :
(a) de 5 % à 60 % d'agent de génération de chaleur inorganique qui génère de la chaleur lorsqu'il est mélangé à l'eau ;
(b) de 0,1 % à 10 % de dérivé polyoxyalkylène comprenant un copolymère polyoxyéthylène/polyoxypropylène ;
(c) de 0,1 % à 30 % d'un composé gras à point de fusion élevé ;
(d) de 0,05 % à 10 % d'une amidoamine de formule générale :
R¹ CONH (CH₂)ₘ N (R²)₂
dans laquelle R¹ est un résidu d'acides gras en C₁₁ à C₂₄, R² est un alkyle en C₁ à C₄, et m est un entier compris entre 1 et 4;
(e) un acide choisi parmi le groupe constitué d'acide ℓ-glutamique, acide lactique, acide chlorhydrique, acide malique, acide succinique, acide acétique, acide fumarique, chlorhydrate d'acide ℓ-glutamique, acide diacétyltartrique, et leurs mélanges, à un niveau tel que le rapport molaire de l'amidoamine sur l'acide est d'environ 1:0,3 à environ 1:1 ; et
(f) un véhicule inerte.

18. Composition de produit de beauté anhydre selon la revendication 14, dans laquelle la composition de conditionnement des cheveux anhydre comprenant en poids :
(a) de 5 % à 60 % de l'agent de génération de chaleur inorganique qui génère de la chaleur en le mélangeant à l'eau ;
(b) de 10 % à 90 % du dérivé polyoxyalkylène comprenant un ester glycérique polyoxyéthylène ;
(c) de 0,1 % à 30 % d'un composé gras à point de fusion élevé;
(d) de 0,05 % à environ 10 % d'une amidoamine de formule générale suivante :
R¹ CONH (CH₂)ₘ N (R²)₂
dans laquelle R¹ est un résidu d'acides gras en C₁₁ à C₂₄, R² est un alkyle en C₁ à C₄, et m est un entier compris entre 1 et 4 ;
(e) un acide choisi parmi le groupe constitué d'acide ℓ-glutamique, acide lactique, acide chlorhydrique, acide malique, acide succinique, acide acétique, acide fumarique, chlorhydrate d'acide ℓ-glutamique, acide diacétyltartrique, et leurs mélanges, à un niveau tel que le rapport molaire de l'amidoamine sur l'acide est d'environ 1:0,3 à environ 1:1.

19. Procédé d'utilisation d'une composition de conditionnement des cheveux selon la revendication 14, dans lequel la composition est appliquée sur cheveux humides pour se mélanger avec l'eau restant sur les cheveux.
